# EUROPÄISCHE PATENTSCHRIFT

(11) **EP 1 455 688 B1**
(45) Veröffentlichungstag und Bekanntmachung des Hinweises auf die Patenterteilung: **15.09.2010**
(21) Anmeldenummer: 02792990.0
(22) Anmeldetag: 13.12.2002
(51) Int. Cl.: A61F 2/06

(54) **GEFÄSSPROTHESE, INSBESONDERE ZUM ERSATZ VON HERZNAHEN BEREICHEN DER AORTA**
VESSEL PROSTHESIS, PARTICULARLY FOR THE REPLACEMENT OF AORTA SEGMENTS NEAR THE HEART
PROTHESE DE VAISSEAU SANGUIN EN PARTICULIER POUR REMPLACER DES ZONES AORTIQUES PROCHES DU COEUR

(30) Priorität: 14.12.2001 DE 10162821; 05.09.2002 DE 10242153
(43) Veröffentlichungstag der Anmeldung: 15.09.2004
(73) Patentinhaber: Aesculap AG, 78532 Tuttlingen (DE); Sievers, Hans-Hinrich, Prof. Dr. med., 24119 Kronshagen (DE)
(72) Erfinder: SIEVERS, Hans-Hinrich, 24119 Kronshagen (DE); LIPPOTH, Lisa, 78588 Denkingen (DE)
(74) Vertreter: Patentanwälte Ruff, Wilhelm, Beier, Dauster & Partner
(86) Internationale Anmeldenummer: PCT/EP2002/014177
(87) Internationale Veröffentlichungsnummer: WO 2003/051232

(56) Entgegenhaltungen:
- WO-A-96/32077
- WO-A-03/034948
- FR-A- 2 248 015
- JP-A- 2001 161 726
- US-A- 4 544 599

## Beschreibung

Die Erfindung betrifft eine Gefäßprothese, insbesondere zum Ersatz von herznahen Bereichen der Aorta.

Gefäßprothesen zum Einsatz von Hohlorganen bei Mensch und Tier, insbesondere von Blutgefäßen, sind seit langem bekannt. Sie können sowohl aus textilem (DE-A2 26 13 575, DE-A2 20 09 349, DE-A1 24 61 370) als auch aus nicht textilem Material (vgl. EP-A1 0 106 496, GB-A1 15 06 432) bestehen.

Da die, insbesondere größeren, Blutgefäße über den größten Teil ihrer Länge einen annähernd geradlinigen Verlauf durch den Körper aufweisen, weisen auch die Gefäßprothesen in der Regel die Form von geraden Röhren auf.

Es gibt jedoch auch Gefäße bzw. Gefäßabschnitte, die einen gekrümmten Verlauf aufweisen. So zeigt beispielsweise die Aorta (große Körperschlagader) des Menschen im Brustkorbbereich kurz nach dem Austritt aus dem Herzen einen bogenförmigen Verlauf. Sie wird in diesem Bereich in folgende Abschnitte unterteilt: Die Aorta entspringt aus der linken Herzkammer, mit dem ersten, aufsteigenden, Abschnitt (pars ascendens). Dieser Teil geht über in den Aortenbogen (arcus aortae), welcher wiederum in den absteigenden Teil der Aorta (pars descendens), übergeht. Alle drei Abschnitte verlaufen mehr oder weniger gekrümmt, wobei insbesondere die pars ascendens und am deutlichsten der Aortenbogen betroffen sind. Die pars descendens ist insbesondere am Anfangsteil gekrümmt.

Kommt es nun zu Erkrankungen wie beispielsweise Arteriosklerose oder zu Verletzungen im Bereich der genannten, gekrümmten Aortenabschnitte, so werden in der Herzchirurgie bislang oftmals gerade Gefäßprothesen benutzt, die vom Operateur während der Operation erst in die gewünschte bogenförmige Form gebracht werden. Dies hat für den Operateur und insbesondere für den Patienten gravierende Nachteile. Zum einen ist es für einen Operateur umständlich, eine gerade Gefäßprothese während der Operation erst in die gewünschte Bogenform zu bringen, um diese dann an die natürlichen Gefäßenden anzunähen. Weitaus gravierender sind die Nachteile jedoch für den Patienten, da diese Prothesen im implantierten Zustand Einknickungen zeigen. Diese Einknickungen kommen dadurch zustande, dass sich der künstliche Aortenbogen bei einem Innendruck nach oben hin verlängert, wodurch er seine Bogenform verliert und abknickt.

Diese Abknickungen können Wirbel bilden, den Durchflussquerschnitt verringern und möglicherweise als Thrombus- und Thromboembolieherde wirken. Somit stellen sie für den Patienten eine nicht unerhebliche Gefahr dar.

Es besteht die Möglichkeit vorgekrümmte Gefäßprothesen aus textilem eine Plissierung aufweisendem Rohrmaterial herzustellen. Hierzu können gerade Stücke auf eine U-Form gezogen und thermisch fixiert werden. Diese thermisch vorgeformten Gefäßbögen haben den Vorteil, dass sie für einen Operateur praktischer handhabbar und implantierbar sind, als die oben genannten geraden Gefäßprothesen, welche während der Operation erst in die passende Form gebracht werden müssen. Allerdings zeigen diese thermisch fixierten Gefäßbögen im implantierten Zustand ähnliche Abknickungen, die auch bei den geraden Gefäßprothesen beobachtet werden.

Aus der Monographie Ishemic Heart Disease, 1999, Seite 398, Mosby/London, ISBN 0723429111, ist auch eine Gefäßprothese bekannt, die zur Fixierung der Bogenform an der Innenseite in einer Reihe stehende Einzeistiche aufweist, durch die ein partielles Zusammenraffen der Gefäßprothesenwand an der Bogeninnenseite erreicht wird. Die Fadenenden der Einzelstiche sind jeweils miteinander verknotet. Allerdings weist auch dieser Gefäßbogen Schwächen durch Formveränderungen im implantierten Zustand auf.

Aus der WO 03/034948 A1 ist eine gebogene und plissierte Gefäßprothese bekannt, welche zur Verringerung des Risikos von Abknickungen sich in Längs- und Querrichtung erstreckende Zugbegrenzungen aufweisen kann.

Der Erfindung liegt daher die Aufgabe zugrunde, eine Gefäßprothese zu schaffen, welche die oben genannten Formveränderungen, insbesondere Abknickungen, vermeidet und im implantierten Zustand auch bei Innendruck im Wesentlichen den vollen Durchgangsquerschnitt behält.

Die Aufgabe wird gelöst durch eine Gefäßprothese mit den technischen Merkmalen aus Anspruch 1, insbesondere zum Ersatz von herznahen Bereichen der Aorta, in Form einer flexiblen Röhre mit einer Falten aufweisenden Plissierung, wobei die Röhre als Bogen ausgebildet ist und die Bogenform im nicht implantierten Zustand durch mindestens eine sich in Längsrichtung der Gefäßprothese erstreckende durchgehende Zugbegrenzung und/oder durch eine Vielzahl von sich in Querrichtung erstreckenden Zugbegrenzungen zugfest fixiert ist. Die Falten der Plissierung sind zur Fixierung des Bogens zusammengerafft. Die Raffung erstreckt sich vorzugsweise über einen Teil des Röhrenumfangs, der 10 bis 180°, insbesondere 15 bis 90°, beträgt.

Im Gegensatz zu den oben erwähnten nur thermisch fixierten vorgeformten Gefäßbögen, welche im nicht implantierten Zustand durch Ziehen in eine gerade Form gebracht werden können, ist dies bei der erfindungsgemäßen Gefäßprothese nicht möglich.

Ein Vergleich zwischen der bekannten Gefäßprothese mit den Einzelstichen an der Bogeninnenseite und der erfindungsgemäßen Gefäßprothese zeigt, dass die erfindungsgemäße Gefäßprothese ach gegenüber dieser bekannten Gefäßprothese wesentlich formstabiler ist. Die zugfeste Fixierung, die vorzugsweise an der Bogeninnenseite vorgesehen bzw. ausgebildet ist, bewirkt im implantierten Zustand eine hohe Formstabilität, insbesondere im Querschnitt. Durch das Zusammenraffen der Falten der Plissierung zur Fixierung des Bogens über einen Teil des Röhrenumfangs kommt es zu einer deutlichen Erhöhung der Stabilität der Bogenform. Dies bewirkt wiederum im implantierten Zustand eine hohe Formstabilität insbesondere im Querschnitt. Es kommt im Gegensatz zu den oben beschriebenen Gefäßprothesen des Standes der Technik nicht zu einer Torsion oder Erweiterung des Bogens, wobei auch die Entstehung von Abknickungen vermieden wird.

Bei einer besonders bevorzugten Ausführungsform der erfindungsgemäßen Gefäßprothese ist die Röhre als thermisch vorfixierter Bogen ausgebildet. Diese zusätzliche thermische Vorfixierung bewirkt eine weitere Erhöhung der Stabilität der Bogenform. Zudem ist die Raffung an einem thermisch vorfixierten Bogen einfacher vorzunehmen.

Es ist bevorzugt, dass die erfindungsgemäße Gefäßprothese eine poröse Wandung aufweist, da dies das Einwachsen von Gewebe und kleinen Blutgefäßen fördert. Besonders bevorzugt ist es, dass die Gefäßprothese aus textilem Material besteht und insbesondere gewirkt oder gewebt ist. Besonders bevorzugt ist es, dass die Gefäßprothese, insbesondere auf der Außenwandung, mit einem natürlichen oder synthetischen resorbierbaren Material beschichtet ist. Hierfür eignet sich beispielsweise Gelatine, insbesondere vernetzte Gelatine. Dieses Material wird nach der Implantation langsam resorbiert und durch körpereigenes Gewebe ersetzt.

Die erfindungsgemäße Gefäßprothese weist mindestens im gebogenen Bereich eine faltenförmige Plissierung in der Prothesenwand auf. Die Plissierung kann wendelförmig oder als geschlossene Ringe ausgebildet sein. Allgemein bewirkt eine Plissierung eine Elastizität und Durchmesserstabilität der Gefäßprothese.

Mit Vorteil besitzt die Gefäßprothese 3 bis 12, vorzugsweise 4 bis 8, insbesondere 6, Falten pro cm Prothesenlänge. Mit Vorteil besitzen die Falten der Plissierung eine Tiefe von 0,5 bis 2,5, vorzugsweise 1 bis 1,5 mm, die insbesondere bei Prothesen mit größerem Durchmesser größer ist als bei Prothesen mit kleinerem Durchmesser. In der Regel beträgt die Faltentiefe mindestens 1 mm.

Mit Vorteil schließen die Falten an der Bogenaußenseite von ihrer Spitze aus einen Winkel von 70° bis 110°, insbesondere von ca. 90°, ein.

Zur Erreichung der oben genannten zugfesten Fixierung des Bogens sind die Falten der Plissierung vorzugsweise mindestens an der Bogeninnenseite in einander angenäherter Stellung fixiert. Zur Erreichung einer Verdrehungsstabilität der Gefäßprothese kann diese auch eine seitliche Fixierung der Falten der Plissierung aufweisen. Mit Vorteil sind die Faltenabstände an der Bogeninnenseite am engsten und erweitern sich in Richtung der Seiten des Bogens. Bevorzugt sind die Falten der Plissierung an der Bogeninnenseite so angenähert, dass sie an der Bogeninnenseite aneinander liegen.

Bei einer besonders bevorzugten Ausführungsform der erfindungsgemäßen Gefäßprothese sind die Falten der Plissierung zur Bildung und Fixierung eines Bogens über einen Teil des Röhrenumfanges bleibend zusammengerafft, insbesondere zusammengenäht, der 10 bis 180°, insbesondere 15 bis 90° entlang des Röhrenumfanges, beträgt. Das Ausmaß der Krümmung wird durch die Anzahl der Nähte und den Stichabstand bestimmt. Vorzugsweise ist jede Falte der Plissierung mit der nächsten verbunden, insbesondere zusammengenäht, was eine hohe Formstabilität gewährleistet. Vorzugsweise umfasst jede Naht eine Vielzahl von Stichen.

Die Gefäßprothese weist als Fixierungsmittel mindestens eine Naht aus mindestens einem Faden auf, die mindestens acht Durchstechungen der Prothesenwand umfasst. Die Falten der Plissierung sind zur Fixierung des Bogens mindestens über einen Teil des Röhrenumfanges einander angenähert, wobei sie sich insbesondere gegenseitig berühren. In der vorliegenden Beschreibung wird eine Naht als solche definiert, die mindestens acht Durchstechungen aufweist und aus einem zusammenhängenden Faden mit Fadenanfang und Fadenende gebildet ist. Unter einer Durchstechung wird in diesem Zusammenhang eine Durchtrittsstelle des Nähfadens durch die Prothesenwand verstanden. Fadenanfang und Fadenende sind vorzugsweise räumlich voneinander getrennt. Eine Naht kann einen Stich, mehrere Stiche oder eine Vielzahl von Stichen aufweisen, beispielsweise Kreuzstiche und/oder Überschlagstiche.

Bei einer Ausführungsform der Erfindung weist die erfindungsgemäße Gefäßprothese mindestens eine Naht auf, welche eine Vielzahl, insbesondere ca. 20 bis 250, von Durchstechuhgen aufweist, wobei sich die Naht vorzugsweise in Längs- und/oder in Umfangsrichtung der Prothese erstreckt. Erstreckt sich die Naht in Längsrichtung der

Prothese, so reicht bereits eine Naht aus, um eine genügend hohe Formstabilität zu erreichen. Durch die Vielzahl zusammenhängender Durchstechungen wird eine hohe Formstabilität erreicht. Vorteilhafterweise ist die erfindungsgemäße Gefäßprothese mindestens an der Bogeninnenseite zugfest ausgebildet. Eine Fixierung über eine Umfangsbreite von 10° bringt schon gute Ergebnisse. Eine Fixierung über eine Breite von 15° bis 30° des Röhrenumfanges bringt eine erhöhte Formstabilität. Zur Erreichung der oben genannten zugfesten Fixierung des Bogens sind die Falten der Plissierung vorzugsweise mindestens an der Bogeninnenseite in einander angenäherter Stellung fixiert. Ist nur eine Naht vorgesehen, dann hat diese vorzugsweise eine Breite von mindestens 10° des Bogenumfangs. Zur Erreichung einer weiteren Verdrehungsstabilität der Gefäßprothese kann diese auch seitliche Fixierungen der Falten der Plissierung aufweisen. Die Nähte können sich in Längs- und/oder Querrichtung der Prothese erstrecken. Hierzu können verbreiterte Nähte oder mehrere parallele Nähte vorgesehen sein. Mit Vorteil sind die Faltenabstände an der Bogeninnenseite am engsten und erweitern sich in Richtung der Seiten des Bogens. Bevorzugt sind die Falten der Plissierung an der Bogeninnenseite so angenähert, dass sie an der Bogeninnenseite mindestens teilweise aneinander liegen.

Bei einer Ausführungsform der erfindungsgemäßen Prothese ist mindestens eine durchgehende Längsnaht insbesondere auf einer zentralen Linie der Bogeninnenseite vorgesehen, wobei sich der Faden über eine Vielzahl von Stichen von einem Ende der Raffung zum anderen erstreckt. Dadurch kommt es zur Zusammenraffung aller Falten entlang einer Linie; d.h. zu einem lückenlosen Zusammenraffen der Falten. Dies ist, wie bereits oben erwähnt, bei den Gefäßprothesen des Standes der Technik nicht der Fall. Zwischenabbindungen, insbesondere Zwischenknoten, zwischen den Falten erlauben ein Verkürzen der Prothese oder ein Entfernen einzelner Stiche, ohne dass die Naht als Ganzes aufgeht.

Bei einer bevorzugten Variante der erfindungsgemäßen Gefäßprothese weist mindestens eine Naht Kreuzstiche auf, besteht insbesondere aus solchen. Die Stiche können Einfach-, Doppel-, oder Mehrfachkreuzstiche sein. Mit Vorteil weist eine Naht, insbesondere eine Längsnaht, zwischen ca. 10 und 40, vorzugsweise zwischen 20 und 30, Stiche, vorzugsweise Kreuzstiche, pro Naht auf. Quernähte sind in der Regel kürzer, da sie maximal 180° des Prothesenumfangs erfassen. Ihre Stichzahl liegt bei ca. 10 bis 20. Kreuzstiche haben sowohl eine Längsals auch eine Querausdehnung.

Besonders bevorzugt ist es, dass sich Nähte, insbesondere Kreuzstiche aufweisende Nähte, in Umfangsrichtung der Prothese erstrecken, wobei sich vorzugsweise zahlreiche parallele Nähte in Umfangsrichtung erstrecken. Sie können sich über eine Breite der Prothese erstrecken, die ca. 5 bis 50 %, insbesondere ca. 10 bis 40 %, des Gesamtumfanges der Röhre entspricht. Die sich in Umfangsrichtung erstreckenden Nähte können durch einen durchgehenden Faden miteinander zu einer zusammenhängenden Naht verbunden sein. Es können sich also Nähte mit Kreuzstichen in Längs- und/oder Umfangsrichtung erstrecken. Durch dieses Erstrecken der Nähte in Umfangsrichtung der Prothese wird eine starke Verbreiterung der Fixierung erreicht, wobei auch der Zusammenraffungsgrad der Falten gesteigert wird. Im Vergleich zu einer Gefäßprothese, bei der jeweils nur einzelne Stellen an der Protheseninnenseite punktweise zusammengerafft sind, kommt es hier zu einer flächenartigen, insbesondere zusammenhängenden Ausdehnung der Zusammenraffungsbereiche. Dies bringt eine starke Erhöhung der Formstabilität mit sich.

Bei einer weiteren Ausführungsform der erfindungsgemäßen Prothese weist mindestens eine Naht Überschlagstiche auf, besteht insbesondere aus solchen. Vorzugsweise erstrecken sich die Nähte, insbesondere die Nähte, die aus Überschlagstichen bestehen, in Umfangsrichtung der Prothese. Es können mehrere Nähte, vorzugsweise parallel, nebeneinander sich in Umfangsrichtung erstreckend angeordnet sein. Diese Nähte können entweder frei oder miteinander verbunden vorliegen. Auch ist es möglich, dass mehrere Nähte mit kleinerer Stichzahl hintereinander in Umfangsrichtung angeordnet sind. Bei einer besonders bevorzugten Ausführungsform sind Nähte mit kleinen Stichzahlen sowohl hintereinander als auch nebeneinander angeordnet, die sich in Umfangsrichtung erstrecken. Vorzugsweise weisen die Nähte, die nebeneinander angeordnet sind, gleiche Stichzahl auf. Diese Stichzahl kann zwischen 1 und 10, vorzugsweise zwischen 1 und 3, liegen.

Bei einer weiteren bevorzugten Ausführungsform weist mindestens eine Naht nur Durchstiche auf, besteht insbesondere aus solchen. Diese Nahtvariante ist sehr einfach herstellbar. Die Naht weist hierbei mindestens acht, besonders bevorzugt mehr als acht Durchstechungen auf. Bei mehr als acht Durchstechungen können die Fäden zwischen einzelnen Durchstechungen einzeln abgebunden, insbesondere verknotet, sein. Mit Vorteil weist mindestens eine Naht mindestens einen, die Falten der Plissierung durchdringenden, sich über die gesamte Länge der Raffung erstreckenden, Faden auf. Die Fadenenden sind vorzugsweise einzeln abgebunden, insbesondere geknotet. Mit Vorteil weist der Faden zwischen den Fadenenden, insbesondere alle 1-2 Plissierfalten, Abbindungen auf. Eine solche Naht hat den Vorteil, dass sie sehr einfach herstellbar ist.

Die Fadenenden einer Naht sind in einem Abstand zueinander angeordnet. Vorzugsweise sind die einzelnen Fadenenden jeweils für sich abgebunden, insbesondere geknotet.

Längsnähte weisen zwischen den Fadenenden, insbesondere alle 2-3 Plissierfalten, Abbindungen, insbesondere Knoten, die vorzugsweise frei von Fadenenden sind, auf. Diese Abbindungen haben den großen Vorteil, dass trotz durchgehendem Faden, es beim Durchtrennen des Fadens beispielsweise beim Zuschneiden der Gefäßprothese durch den Operateur, nicht zu einem Aufgehen der gesamten Naht kommt. Der Faden kann sich von der Durchtrennungsstelle aus höchstens bis zur nächsten Abbindung von der Gefäßprothese lösen. Vorzugsweise liegen die Abbindungen zwischen den Stichen. Besonders bevorzugt folgt nach jedem Stich eine Abbindung.

Mit Vorteil sind, insbesondere durch eine Quernaht, jeweils zwei bis sechs, insbesondere zwei bis drei, Falten zusammengerafft.

Bei einer bevorzugten Ausführungsform der erfindungsgemäßen Gefäßprothese weist diese mehrere, insbesondere zwei, vorzugsweise im Wesentlichen parallele Längsnähte auf, die sich über eine Vielzahl von Plissierungsfalten erstrecken. Dies bringt, ähnlich wie bei der oben genannten Ausführungsform, bei der sich die Stiche in Umfangsrichtung der Prothese erstrecken, eine Zusammenraffung der Falten der Plissierung über einen größeren Bereich in Umfangsrichtung. Vorzugsweise sind die parallelen Nähte achsensymmetrisch zu einer im Zentrum des Bogeninneren verlaufenden Linie angeordnet. Längsnähte können mit Nähten in Umfangsrichtung der Prothese kombiniert sein.

Besonders bevorzugt ist es, dass die Nähte an der Innenwand der Prothese im wesentlichen nicht offen liegen, insbesondere die Innenkanten der Plissierungsfalten frei von Fadenmaterial sind. Die Nähte verlaufen vorzugsweise nur durch die Wände der Plissierung und gegebenenfalls über deren Außenkanten. Dadurch haben die Nähte nur sehr wenig Blutkontakt, was beispielsweise die Gefahr einer Thrombose herabsetzt.

Bei einer weiteren Ausführungsform der Gefäßprothese sind die Falten der Plissierung zur Fixierung des Bogens an der Bogeninnenseite durch mindestens einen aufgebrachten, insbesondere aus Kunststoff bestehenden, Streifen in einander angenäherter Stellung fixiert. Es ist auch möglich, dass solche Streifen auch seitlich aufgebracht sind, um eine Verdrehungsstabilität zu erreichen. Mit Vorteil sind die genannten Streifen mindestens teilweise, insbesondere nur, auf die Faltenaußenkanten bzw. Faltenberge aufgebracht. Dies bewirkt wiederum, dass in den Faltentälern ein problemloses Einwachsen von Gewebe und kleinen Blutgefäßen möglich ist, da an diesen Stellen die Poren nicht durch den Streifen abgedeckt sind. Bei einer bevorzugten Ausführungsform sind die genannten Streifen auf die Gefäßprothese aufgeklebt und/oder aufgenäht.

Neben den oben genannten bevorzugten Ausführungsformen, bei denen die Falten der Plissierung an der Bogeninnenseite durch Zusammennähen bzw. durch Aufkleben oder Aufnähen eines Streifens in einander angenäherter Stellung fixiert sind, ist es auch möglich, dass die Falten der Plissierung an der Bogeninnenseite zusätzlich durch Schrumpfung in einander angenäherter Stellung fixiert sind. Zu diesem Zwecke ist es möglich, mindestens teilweise ein hochschrumpfungsfähiges Material, insbesondere ein hochschrumpfungsfähiges Garn, in die Gefäßprothesenwand zu integrieren. Die erfindungsgemäßen Gefäßprothesen weisen vorzugsweise einen Durchmesser von 15 bis 50 mm, insbesondere 20 bis 40 mm, auf. Der Durchmesser wird in der Regel so gewählt, dass er dem natürlichen Durchmesser des ersetzten Gefäßes entspricht. Es ist auch möglich, eine erfindungsgemäße Gefäßprothese einem nicht erwachsenen Menschen zu transplantieren, wobei in diesem Fall jedoch von Zeit zu Zeit die Gefäßprothese ausgewechselt werden muss, um den Durchmesser an den stegenden Durchmesser des, an Größe zunehmenden, Gefäßes anzupassen.

Mit Vorteil beträgt der Radius des Gefäßbogens in der Prothesenlängsmittelachse 3 bis 6 cm, insbesondere 4 bis 5 cm. Auch diese Werte hängen von den anatomischen Verhältnissen des Patienten ab.

In der Regel schließt sich an die Krümmung des Bogens an mindestens einer Seite der erfindungsgemäßen Gefäßprothese, vorzugsweise an beiden Seiten, ein geradlinig verlaufender Prothesenabschnitt an. Im Falle der Aortenprothese entsprechen diese geradlinig verlaufenden Abschnitte mindestens Teilen der pars ascendens und/oder pars descendens. Diese geradlinig verlaufenden Abschnitte können auch unplissiert sein.

Mit Vorteil schließt der Bogen der Prothese einen Winkel von 120 bis 330, insbesondere 180 bis 270°, ein. Vorzugsweise besitzt die erfindungsgemäße Gefäßprothese eine Konizität, die der Konizität der natürlichen Aorta im wesentlichen entspricht.

Alle größeren Blutgefäße im menschlichen Körper weisen Abzweigungen auf. So treten aus der Aorta im Bereich des Aortenbogens drei abzweigende Blutgefäße aus (Truncus brachiocephalicus, a. carotis communis sin., a. subclavia sin.). Um diesen natürlichen Verhältnissen gerecht zu werden, weist eine bevorzugte Ausführungsform der erfindungsgemäßen Gefäßprothese den genannten abzweigenden Gefäßen entsprechende, aus der Gefäßprothese austretende, künstliche Abzweigungen auf. Diese können, je nach Bedarf und anatomischen Verhältnissen, unterschiedliche Durchmesser und Länge aufweisen. Die Länge dieser abzweigenden Prothesenabschnitte hängt insbesondere vom Ausmaß der Erkrankung bzw. der Verletzung der betreffenden Gefäße ab.

Ist beispielsweise nur der Aortenbogen von einer Krankheit bzw. Verletzung betroffen, so ist es auch möglich, dass die natürlichen Gefäßabzweigungen direkt auf Öffnungen in der Gefäßprothese aufgenäht werden, und die Gefäßprothese keine künstlichen Abzweigungen aufweist. Die genannten Öffnungen könnten entweder bereits in der Prothese enthalten sein oder erst während der Operation vom Operateur aus der Prothesenwand ausgeschnitten werden.

Zudem kann die Prothese auch mit einem Ansatz für die Reperfusion mittels der Herz-Lungenmaschine versehen sein.

Die erfindungsgemäße Gefäßprothese kann auch Bifurkationen (Abzweigungen) sowie Bulbi (bauchartige Erweiterungen) aufweisen.

Weitere Einzelheiten und Merkmale der Erfindung ergeben sich aus der nachfolgenden Beschreibung eines bevorzugten Ausführungsbeispiels in Verbindung mit den Unteransprüchen. Hierbei können die jeweiligen Merkmale für sich allein oder zu mehreren in Kombination miteinander verwirklicht sein.

### Figurenbeschreibung

In den Zeichnungen zeigen:

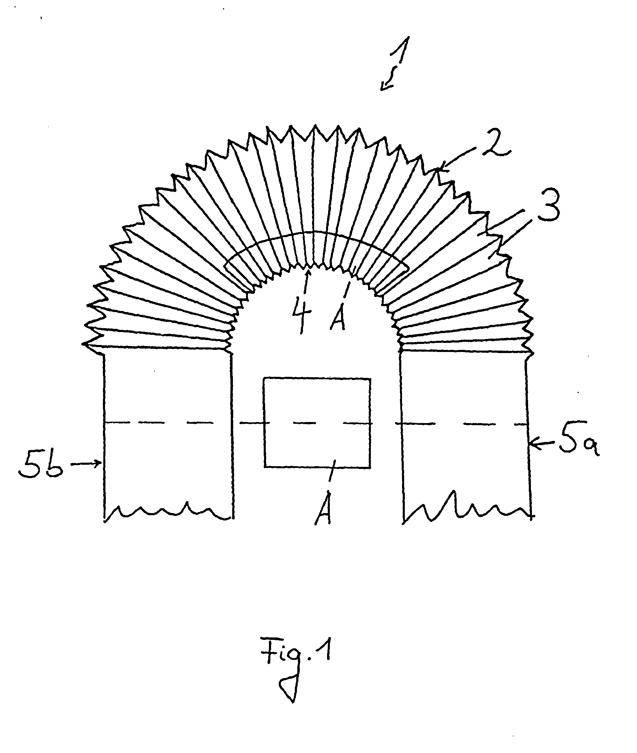

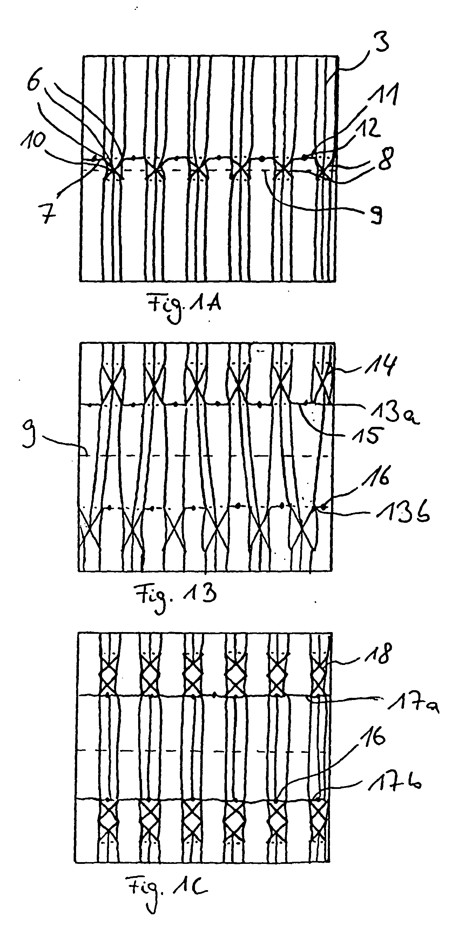

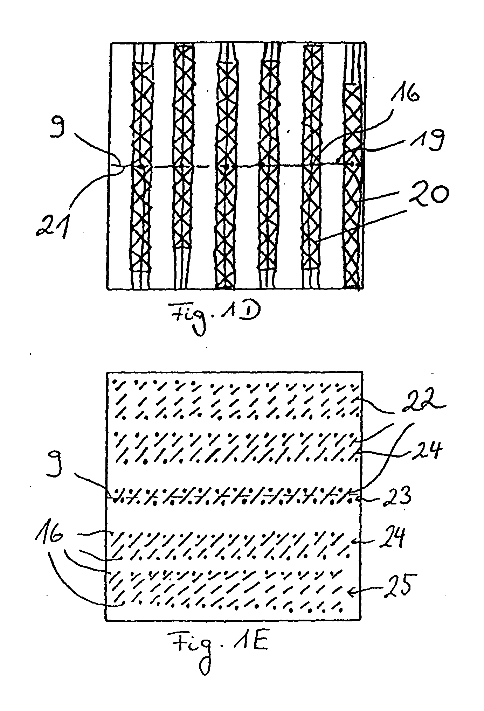

- Figur 1:: Seitenansicht einer erfindungsgemäßen Gefäßprothese aus textilem Material mit genähter Fixierung,
- Figur 1 a:: Draufsicht auf einen Ausschnitt A aus dem zentralen Bereich des Bogenscheitels einer erfindungsgemäßen Gefäßprothese mit einer Naht aus Einzelkreuzstichen.
- Figur 1b:: Draufsicht auf einen Ausschnitt aus dem zentralen Bereich des Bogenscheitels einer erfindungsgemäßen Gefäßprothese mit zwei parallel verlaufenden Nähten aus Einzelkreuzstichen.
- Figur 1c:: Draufsicht auf einen Ausschnitt aus dem zentralen Bereich des Bogenscheitels einer erfindungsgemäßen Gefäßprothese mit zwei parallel verlaufenden Nähten aus Doppelkreuzstichen.
- Figur 1 d:: Draufsicht auf einen Ausschnitt aus dem zentralen Bereich des Bogenscheitels einer erfindungsgemäßen Gefäßprothese mit einer Naht aus Mehrfachkreuzstichen.
- Figur 1 e:: Draufsicht auf einen Ausschnitt aus dem zentralen Bereich des Bogenscheitels einer erfindungsgemäßen Gefäßprothese mit einer Vielzahl von Nähten aus Überschlagstichen.
- Figur 2:: Vergrößerte Seitenansicht einer erfindungsgemäßen Gefäßprothese mit genähter Fixierung.
- Figur 3:: Seitenansicht einer erfindungsgemäßen Gefäßprothese aus textilem Material mit Fixierung durch aufgeklebten Kunststoffstreifen.

Figur 1 zeigt eine erfindungsgemäße bogenförmige Gefäßprothese 1 aus einem textilen Material mit genähter Fixierung. Hierbei kommt eine Vielzahl verschiedener Nähte in Frage. Diese sind in den Figuren 1A bis 1 E schematisch dargestellt. Diese Prothese ist geeignet zum Ersatz von gebogenen Gefäßen bzw. Gefäßabschnitten, insbesondere zum Ersatz des Aortenbogens und Teilen der pars ascendens und pars descendens. Der Bogen der rohrförmigen Gefäßprothese schließt einen Winkel von 180° ein. Die Prothese hat einen Umfang von ca. 7 cm. In den Figuren 1A bis 1D sind jeweils nur die Faltenberge zu sehen.

Im Bereich des gebogenen Abschnitts 2 der gezeigten Gefäßprothese weist diese eine faltenförmige Plissierung in der Prothesenwand auf, wobei die Falten 3 dieser Plissierung als geschlossene Ringe ausgebildet sind. Die Falten 3 der Plissierung sind an der Bogeninnenseite 4 zur Fixierung des Bogens in einander angenäherter Stellung fixiert. Diese Fixierung ist im vorliegenden Ausführungsbeispiel durch Zusammennähung der Falten über einen Teil des Röhrenumfangs längs der Bogeninnenseite vorgenommen. Details über alternative Nähte finden sich in den Beschreibungen der Figuren 1a bis 1 e. Die Faltenabstände an der Bogeninnenseite 4 sind am engsten und erweitern sich in Richtung der Außenseite des Aortenbogens 2. An den gebogenen und plissierten Abschnitt 2 der Gefäßprothese 1 schließen sich an beiden Seiten des Bogens jeweils ein gerader Abschnitt 5a und 5b an. Diese geraden Abschnitte 5 weisen keine Plissierungen auf.

Figur 1A zeigt einen Ausschnitt A aus dem zentralen Bereich des Bogeninnenscheitels einer erfindungsgemäßen Gefäßprothese. Diese Gefäßprothese weist eine in Längsrichtung verlaufende Naht 7 auf, die eine Vielzahl von Einfachkreuzstichen 8 aufweist. Diese Naht verläuft so, dass eine gedachte zentrale Linie 9 des Bogeninneren im wesentlichen durch die Schnittpunkte 10 der Einfachkreuzstiche verläuft. Die Naht 7 weist eine, die Stiche 8 verbindende Grundlinie 11 auf, entlang der das Fadenmaterial entlang der gesamten Raffung der Plissierung verläuft und die Kreuzstiche untereinander verbindet und beieinander hält. Zudem weist die Naht 7 Zwischenabbindungen des Fadens in Form von Knoten 12 auf, welche auf der Grundlinie liegen. Dabei folgt nach jedem Stich ein Knoten. Die Gefäßprothese weist ca. 3 Stiche pro cm auf. Insgesamt sind 23 Stiche über die Länge des Bogens vorhanden. Durch jeden Stich werden drei Falten der Plissierung 3 zusammengerafft. Somit umfasst jeder Stich mindestens 12 Durchstechungen (pro Falte 4 Durchstechungen der Prothesenwand). Die Falten sind über einen Teil des Röhrenumfanges zusammengerafft, der ca. 15° beträgt. Die ganze Naht besteht aus einem zusammenhängenden Faden.

Figur 1B zeigt einen Ausschnitt aus dem zentralen Bereich des Bogeninnenscheitels 9 einer erfindungsgemäßen Gefäßprothese. Bei dieser Ausführungsform verlaufen zwei Nähte 13a, 13b im wesentlichen parallel in Längsrichtung. Die beiden Nähte weisen Kreuzstiche 14 auf, die durch eine Grundlinie 15 aus Fadenmaterial miteinander verbunden sind, das die einzelnen Stiche beieinander hält und somit ebenfalls zur Raffung beiträgt. Das Fadenmaterial auf der Grundlinie weist Knoten 16 auf, wobei nach jedem Stich ein Knoten folgt. Die Stiche erstrecken sich ca. 6mm in Umfangsrichtung der Prothese. Die beiden Nähte sind annähernd achsensymmetrisch parallel zur Linie 9 angeordnet. Jede Naht weist zwischen 20 und 25 Stiche auf. Jede Naht 13a und 13b weist ca. 3 bis 4 Kreuzstiche pro cm Prothesenlänge auf. Pro Kreuzstich werden entweder zwei oder drei Falten der Plissierung zusammengerafft. Wie in Figur 1a umfasst jeder Stich mindestens 12 Durchstechungen. Die Stiche der beiden Nähte liegen sich nicht direkt gegenüber, sondern sind jeweils um eine Plissierfalte gegeneinander versetzt. Durch den äußeren Rand der ersten Naht und dem äußeren Rand der zweiten Naht werden ca. 90° des Röhrenumfangs eingeschlossen. Zwischen den beiden Nähten im Innenbereich des Bogens liegen die Falten eng aneinander.

Figur 1C zeigt einen Ausschnitt aus dem zentralen Bereich des Bogenscheitels einer erfindungsgemäßen Gefäßprothese. Diese Ausführungsform weist, ebenso wie die Ausführungsform in Figur 1b, zwei parallele in Längsrichtung verlaufende Nähte 17a und 17b auf. Die Ausführungsform in 1C gleicht der in Figur 1B mit folgenden Ausnahmen: Die Stiche sind Doppelkreuzstiche 18; die Stiche der beiden Nähte liegen sich gegenüber, wodurch die sich gegenüber liegenden Stiche die gleichen Falten zusammenraffen (im Bogeninneren jeweils 3 Falten und im Außenbereich bei den Bogenenden jeweils 2 Falten); die Knoten liegen größtenteils im Bereich der Stiche. Jeder Doppelkreuzstich, der 3 Falten zusammenrafft, umfasst 16 Durchstechungen (je 6 Durchstechungen der beiden äußeren Falten und 4 Durchstechungen der mittleren Falte). Jeder Doppelkreuzstich, der 2 Falten zusammenrafft, umfasst mindestens 18 Durchstechungen.

Figur 1D zeigt einen Ausschnitt aus dem zentralen Bereich des Bogenscheitels einer erfindungsgemäßen Gefäßprothese. Diese Ausführungsform weist eine Naht 19 auf, welche zahlreiche parallele Mehrfachkreuzstiche 20 besitzt. Diese Mehrfachkreuzstiche weisen eine unterschiedliche Zahl an Einzelkreuzstichen auf. Diese Zahl bewegt sich zwischen 11 und 16 Stichen. Die Mehrfachkreuzstiche treten links und rechts über die Grundlinie 21 hinaus und erstrecken sich in Umfangsrichtung der Gefäßprothese. Dabei raffen sie die Falten der Plissierung über einen Teil des Röhrenumfanges zusammen, der zwischen 100 und 160° beträgt. Auf der Grundlinie verläuft annähernd in der zentralen Linie 9 ein Faden, der die einzelnen Quernähte miteinander verbindet und jeweils ein Teil des durchgehenden die Nähte bildenden Fadenmaterials ist. Durch die Längsverbindung der einzelnen Quernähte wird der Gesamtkombination der Nähte ein flächenhafter Charakter verliehen. Pro Mehrfachkreuzstich werden im zentralen Bereich des Bogens (Scheitelbereich) 3 Falten pro Stich zusammengerafft, in den Außenbereichen des Bogens werden 2 Falten pro Mehrfachkreuzstich zusammengerafft. Die einzelnen Mehrfachkreuzstiche verlaufen annähernd parallel zueinander. Die vorliegende Ausführungsform weist 24 Mehrfachkreuzstiche auf und weist ca. 4 Mehrfachkreuzstiche pro cm Prothesenlänge auf. Auch weist diese Ausführungsform Knoten 16 auf, die sich im wesentlichen im Bereich der Mehrfachkreuzstiche befinden. Es sind mindestens so viel Zwischenknoten wie Mehrfachkreuzstiche vorhanden.

Figur 1 E zeigt einen Ausschnitt aus dem zentralen Bereich des Bogenscheitels einer erfindungsgemäßen Gefäßprothese. Im Gegensatz zu den bisher beschriebenen Ausführungsformen verlaufen mehrere parallele Nähte in Längsrichtung der Prothese, nämlich eine entlang dem Innenscheitel 9 und jeweils zwei Nähte rechts und links davon. Die Längsnähte haben eine Breite von mindestens zwei, vorzugsweise zwei bis vier Stichen und sind in Längsrichtung in eine Vielzahl von einzelnen aneinander gereihten Quernähten unterteilt. Die Nähte dieser Ausführungsform weisen Überschlagstiche 22 auf. Die Fäden der Einzelnähte weisen an ihren Enden Knoten 16 auf. Entlang der zentralen Mittellinie 9 befindet sich eine Reihe von Einzelnähten 23 mit Einfachüberschlagstichen. Diese Einzelnähte 23 sind untereinander nicht verbunden. In beiden Richtungen in Umfangsrichtung der Gefäßprothese folgt nach jeder Einzelnaht 23 mit Einfachüberschlagstich in einem Abstand von ca. 4 mm jeweils eine Naht 24, welche aus zwei Überschlagstichen besteht. Diese Einzelnähte erstrecken sich in Umfangsrichtung der Gefäßprothese. Die Einzelnähte 23 und die Nähte 24 mit zwei Überschlagstichen befinden sich auf Reihen längs der Prothese. Die Fäden der genannten Einzelnähte 24 weisen an ihren Enden jeweils Knoten 16 auf. Auf diese Nähte 24 mit zwei Überschlagstichen folgen in Umfangsrichtung in einem Abstand von ca. 7 mm weitere Einzelnähte 25, welche allesamt drei Überschlagstiche aufweisen. Auch die Fäden dieser Einzelnähte weisen an ihren Enden Knoten 16 auf. Die Einzelnähte 25 liegen annähernd auf einer Linie in Umfangsrichtung mit je einer Einzelnaht 24 mit zwei Überschlagstichen und einer Naht 23 mit einem Einfachüberschlagstich. Es liegen also jeweils fünf Nähte im wesentlichen hintereinander in Umfangsrichtung (zwei Nähte mit drei Überschlagstichen, zwei Nähte mit zwei Überschlagstichen und eine Naht mit Einfachüberschlagstich). Auch die Einzelnähte 25 liegen in Längsreihen. Durch die auf Linien in Umfangsrichtung und in Reihen in Längsrichtung hintereinander liegenden Einzelnähte werden die Falten der Plissierung flächig zusammengerafft. Nähte mit Einfachüberschlagstichen umfassen 8 Durchstechungen, Nähte mit zwei Überschlagstichen umfassen 12 Durchstechungen und Nähte mit drei Überschlagstichen umfassen 16 Durchstechungen. Die Falten sind der Übersichtlichkeit halber in der vorliegenden Figur nicht eingezeichnet. Dabei sind die Falten der Plissierung zur Fixierung des Bogens über einen Teil des Röhrenumfanges zusammengerafft, der annähernd 180° beträgt. Diese Ausführungsform weist in Längsrichtung ca. 30 Einfachüberschlagstiche auf und damit ca. 60 Nähte mit zwei Überschlagstichen (jeweils 30 nebeneinander in Längsrichtung stehend) und ca. 60 Nähte mit drei Überschlagstichen auf.

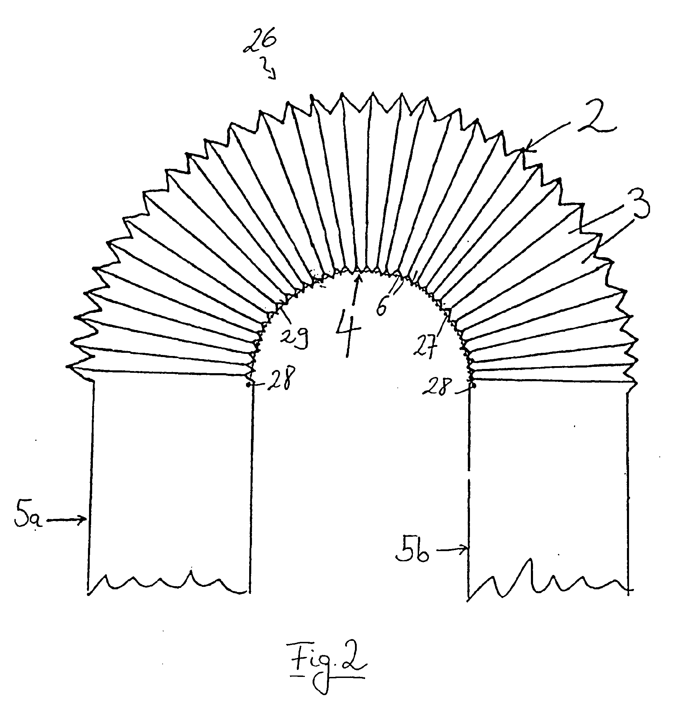

Auch Figur 2 zeigt eine erfindungsgemäße Gefäßprothese 26 aus einem textilen Material mit genähter Fixierung. Bei der hier vorliegenden Naht handelt es sich um eine sehr einfach herstellbare Variante. Diese Naht weist einen in Längsrichtung mittig verlaufenden, die Falten 3 der Plissierung durchdringenden durchgehenden Faden 27 auf. Der Faden 27 weist an seinen Enden Abbindungen 28, z.B. Knoten, auf. Durch diese Abbindungen 28 kommt es zum einen zu einer Raffung an der Bogeninnenseite 4, zum anderen wird durch diese Abbindungen ein Fixieren des Fadens 27 in der Prothese 26 erreicht. Die Naht umfasst zwei Durchstechungen 6 pro Falte. Die Naht, welche eine Längsnaht ist, weist auch zwischen den Fadenenden Abbindungen 29, insbesondere Knoten, auf. Diese Knoten befinden sich in jeder Falte 3 der Plissierung Wie bei den bereits oben beschriebenen Varianten verhindern diese Knoten 29 ein Auftrennen der Naht bei einem möglichen Durchtrennen der selben.

Die genannten Abbindungen (Knoten) 29 können auch etwas weniger zahlreich ausgebildet sein und beispielsweise nur in jeder zweiten oder dritten Falte liegen. Wie bereits oben erwähnt ist der Hauptvorteil dieser Naht die einfache Herstellbarkeit. Es können auch mehrere solcher Nähte parallel nebeneinander liegen.

Auch weist diese Ausführungsform einen gebogenen Teil 2 mit Plissierung auf, an den sich an beiden Seiten des Bogens jeweils ein gerader, unplissierter Abschnitt 5a und 5b anschließt.

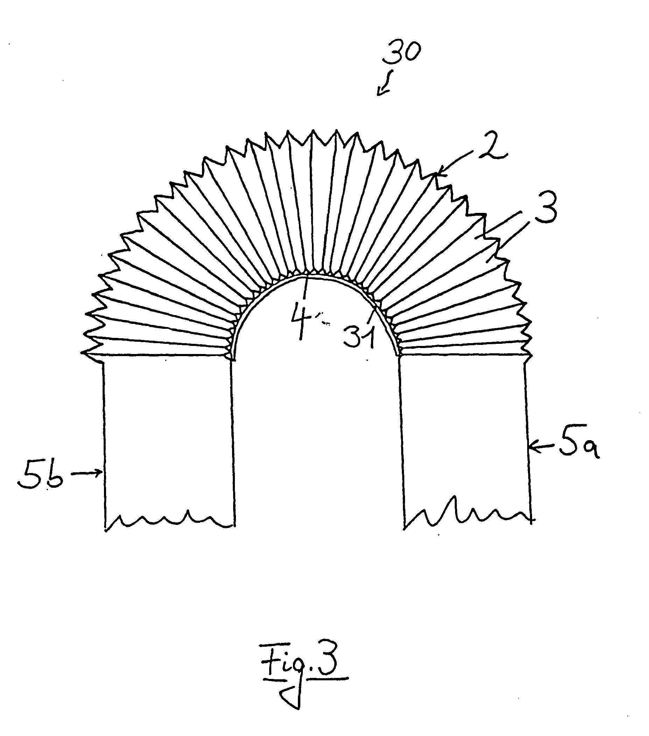

Figur 3 zeigt eine erfindungsgemäße Gefäßprothese 30 ebenfalls aus einem textilen Material. Auch diese Prothese ist geeignet zum Einsatz von gebogenen Prothesen bzw. Gefäßabschnitten, insbesondere zum Ersatz des Aortenbogens und Teilen der pars ascendens und pars descendens. Der Bogen der gezeigten Gefäßprothese schließt einen Winkel von 180° ein. Im Bereich des gebogenen Abschnittes 2 der gezeigten Gefäßprothese weist diese eine faltenförmige Plissierung in der Prothesenwand auf, wobei die Falten 3 dieser Plissierung als geschlossene Ringe ausgebildet sind. Die Falten 3 der Plissierung sind an der Bogeninnenseite 4 zur Fixierung des Bogens in einander angenäherter Stellung fixiert. Diese Fixierung ist im vorliegenden Ausführungsbeispiel durch einen, auf den Faltenspitzen der Bogeninnenseite aufgeklebten zugfesten Kunststoffstreifen 31 vorgenommen. Die Faltenabstände an der Bogeninnenseite 4 sind am engsten und erweitern sich in Richtung der Außenseite des Aortenbogens. An den gebogenen und plissierten Abschnitt 2 der Gefäßprothese 7 schließen sich analog des Ausführungsbeispiels in Figur 1 an beiden Seiten des Bogens jeweils ein gerader Abschnitt 5a und 5b an. Diese geraden Abschnitte 5a und 5b weisen keine Plissierungen auf.

Vergleichsversuche zwischen den erfindungsgemäßen Gefäßprothesen und Gefäßprothesen aus dem Stand der Technik haben gezeigt, dass sich die erfindungsgemäßen Gefäßprothesen bei einer Erhöhung des Innendrucks in ihrer Form stabil bleiben. Dagegen ist bei Gefäßprothesen aus dem Stand der Technik zu beobachten, dass sich diese bei einer Erhöhung des Innendrucks stark verformen und Einknickungen zeigen.

## Patentansprüche

1. Gefäßprothese (1, 26, 30), insbesondere zum Ersatz von herznahen Bereichen der Aorta, in Form einer flexiblen Röhre mit einer Falten (3) aufweisenden Plissierung, wobei die Röhre als Bogen ausgebildet ist und die Bogenform im nicht implantierten Zustand durch mindestens eine sich in Längsrichtung erstreckende durchgehende Zugbegrenzung und/oder durch eine Vielzahl von sich in Querrichtung erstreckende Zugbegrenzungen zugfest fixiert ist, wobei die Falten der Plissierung zur Fixierung des Bogens zusammengerafft sind, **dadurch gekennzeichnet, dass** die Gefäßprothese als Zugbegrenzung mindestens eine Naht (7, 13a, 13b, 17a, 17b, 19, 23, 24, 25) mit mindestens acht Durchstechungen (6) aufweist, wobei sich die mindestens eine Naht in Längs- und/oder Umfangsrichtung der Prothese erstreckt, Fadenenden in einem Abstand zueinander angeordnet sind und Längsnähte zwischen den Fadenenden Zwischenabbindungen aufweisen.

2. Gefäßprothese nach Anspruch 1, **dadurch gekennzeichnet, dass** die plissierte Röhre als thermisch vorfixierter Bogen ausgebildet ist.

3. Gefäßprothese nach einem der Ansprüche 1 oder 2, **dadurch gekennzeichnet, dass** Falten (3) der Plissierung zur Fixierung des Bogens zusammengerafft sind und sich die Raffung über einen Teil des Röhrenumfanges erstreckt, der 10 bis 180° beträgt.

4. Gefäßprothese nach einem der vorhergehenden Ansprüche, **dadurch gekennzeichnet, dass** sie mindestens an der Bogeninnenseite (4) zugfest ausgebildet ist.

5. Gefäßprothese nach einem der vorhergehenden Ansprüche, **dadurch gekennzeichnet, dass** sich mindestens eine Naht (7, 23) auf einer zentralen Linie (9) der Bogeninnenseite (4) befindet.

6. Gefäßprothese nach einem der vorhergehenden Ansprüche, **dadurch gekennzeichnet, dass** mindestens eine Naht (7, 13a, 13b, 17a, 17b, 19) Kreuzstiche (8, 14, 18, 20) aufweist, insbesondere aus solchen besteht.

7. Gefäßprothese nach einem der vorhergehenden Ansprüche, **dadurch gekennzeichnet, dass** mindestens eine Naht, insbesondere mindestens eine Längsnaht, zwischen ca. 1 und 40, vorzugsweise zwischen 20 und 30, Stiche, vorzugsweise Kreuzstiche (8, 14, 18, 20), pro Naht aufweist.

8. Gefäßprothese nach einem der vorhergehenden Ansprüche, **dadurch gekennzeichnet, dass** sich Nähte, insbesondere Kreuzstiche (8, 14, 18, 20) aufweisende Nähte (7, 13a, 13b, 17a, 17b, 19), in Umfangsrichtung der Prothese erstrecken, insbesondere im wesentlichen parallel zueinander, wobei sie sich vorzugsweise über eine Breite erstrecken, die ca. 5 - 50 %, insbesondere ca. 10 bis 40 %, des Gesamtumfanges der Röhre entspricht.

9. Gefäßprothese nach einem der vorhergehenden Ansprüche, **dadurch gekennzeichnet, dass** sich Nähte (7, 13a, 13b, 17a, 17b, 19) mit Kreuzstichen (8, 14, 18, 20) in Längs- und/oder Umfangsrichtung der Prothese erstrecken.

10. Gefäßprothese nach einem der vorhergehenden Ansprüche, **dadurch gekennzeichnet, dass** mindestens eine Naht (23, 24, 25) Überschlagstiche (22) aufweist, insbesondere ausschließlich aus solchen besteht.

11. Gefäßprothese nach einem der vorhergehenden Ansprüche, **dadurch gekennzeichnet, dass** sich Nähte (23, 24, 25) mit Überschlagstichen (22) in Umfangsrichtung der Prothese erstrecken.

12. Gefäßprothese nach einem der vorhergehenden Ansprüche, **dadurch gekennzeichnet, dass** mindestens eine Naht Durchstiche aufweist, insbesondere ausschließlich aus solchen besteht.

13. Gefäßprothese nach einem der vorhergehenden Ansprüche, **dadurch gekennzeichnet, dass** die Innenkanten der Falten (3) der Plissierung frei von Zugbegrenzungen, insbesondere Fäden, sind.

14. Gefäßprothese nach einem der vorhergehenden Ansprüche, **dadurch gekennzeichnet, dass** mindestens eine Naht (7, 13a, 13b, 17a, 17b, 19) mindestens einen, die Falten (3) der Plissierung durchdringenden, sich über die gesamte Länge der Raffung erstreckenden, Faden aufweist.

15. Gefäßprothese nach einem der vorhergehenden Ansprüche, **dadurch gekennzeichnet, dass** die Fadenenden einzeln abgebunden, insbesondere in sich verknotet sind.

16. Gefäßprothese nach einem der vorhergehenden Ansprüche, insbesondere nach Anspruch 15, **dadurch gekennzeichnet, dass** die Längsnähte zwischen den Fadenenden Knoten (12, 16, 28, 29), aufweisen.

17. Gefäßprothese nach einem der vorhergehenden Ansprüche, **dadurch gekennzeichnet, dass** die Längsnähte zwischen den Fadenenden alle ein bis drei Plissierfalten Zwischenabbindungen, insbesondere Knoten (12, 16, 28, 29), aufweisen.

18. Gefäßprothese nach einem der vorhergehenden Ansprüche, **dadurch gekennzeichnet, dass** durch eine Quernaht jeweils 2 bis 6, insbesondere jeweils 2 bis 3, Falten (3) zusammengerafft sind.

19. Gefäßprothese nach einem der vorhergehenden Ansprüche, **dadurch gekennzeichnet, dass** sie mehrere, insbesondere zwei, vorzugsweise im wesentlichen parallele Längsnähte (13a, 13b, 17a, 17b) aufweist, die sich über eine Vielzahl von Plissierungsfalten (3) erstrecken.

20. Gefäßprothese nach einem der vorhergehenden Ansprüche, **dadurch gekennzeichnet, dass** die Falten (3) der Plissierung zur Fixierung des Bogens an der Bogeninnenseite durch mindestens einen daran befestigten zugfesten Streifen (31) in einander angenäherter Stellung fixiert sind.

21. Gefäßprothese nach Anspruch 20, **dadurch gekennzeichnet, dass** der Streifen (31) mindestens auf, insbesondere nur auf, den Faltenspitzen befestigt, insbesondere auf diese aufgeklebt ist.

## Claims

1. Vessel prosthesis (1, 26, 30), particularly for the replacement of aorta segments near the heart, in the form of a pleated flexible tube with folds (3), the tube being configured as an arc, and the arc shape being fixed in an extension-resistant manner, in the non-implanted state, by means of at least one continuous extension limiter running in the longitudinal direction and/or by a multiplicity of extension limiters running in the transverse direction, the folds of the pleating being gathered together for fixing the arc, **characterized in that** the vessel prosthesis has, as extension limiter, at least one seam (7, 13a, 13b, 17a, 17b, 19, 23, 24, 25) with at least eight through-runs (6), the at least one seam running in the longitudinal direction and/or circumferential direction of the prosthesis, thread ends being arranged at a distance from one another and longitudinal seams having, between the thread ends, intermediate ties.

2. Vessel prosthesis according to Claim 1, **characterized in that** the pleated tube is configured as a thermally pre-fixed arc.

3. Vessel prosthesis according to either of Claims 1 and 2, **characterized in that** folds (3) of the pleating are gathered together for fixing the arc, and the gather extends over a part of the tube circumference amounting to 10 to 180°.

4. Vessel prosthesis according to one of the preceding claims, **characterized in that** it is made extension-resistant at least on the inner side (4) of the arc.

5. Vessel prosthesis according to one of the preceding claims, **characterized in that** at least one seam (7, 23) is located on a central line (9) of the inner side (4) of the arc.

6. Vessel prosthesis according to one of the preceding claims, **characterized in that** at least one seam (7, 13a, 13b, 17a, 17b, 19) has cross-stitches (8, 14, 18, 20), and in particular consists of such.

7. Vessel prosthesis according to one of the preceding claims, **characterized in that** at least one seam, in particular at least one longitudinal seam, has between ca. 1 and 40, preferably between 20 and 30, stitches, preferably cross-stitches (8, 14, 18, 20), per seam.

8. Vessel prosthesis according to one of the preceding claims, **characterized in that** seams, in particular seams (7, 13a, 13b, 17a, 17b, 19) having cross-stitches (8, 14, 18, 20), run in the circumferential direction of the prosthesis, in particular substantially parallel to one another, and they preferably extend over a width which corresponds to ca. 5 - 50%, in particular ca. 10 to 40%, of the total circumference of the tube.

9. Vessel prosthesis according to one of the preceding claims, **characterized in that** seams (7, 13a, 13b, 17a, 17b, 19) with cross-stitches (8, 14, 18, 20) run in the longitudinal direction and/or circumferential direction of the prosthesis.

10. Vessel prosthesis according to one of the preceding claims, **characterized in that** at least one seam (23, 24, 25) has ride-over stitches (22), and in particular consists exclusively of such.

11. Vessel prosthesis according to one of the preceding claims, **characterized in that** seams (23, 24, 25) with ride-over stitches (22) run in the circumferential direction of the prosthesis.

12. Vessel prosthesis according to one of the preceding claims, **characterized in that** at least one seam has through-stitches, and in particular consists exclusively of such.

13. Vessel prosthesis according to one of the preceding claims, **characterized in that** the inner edges of the folds (3) of the pleating are free from extension limiters, in particular threads.

14. Vessel prosthesis according to one of the preceding claims, **characterized in that** at least one seam (7, 13a, 13b, 17a, 17b, 19) has at least one thread which passes through the folds (3) of the pleating and runs the entire length of the gather.

15. Vessel prosthesis according to one of the preceding claims, **characterized in that** the thread ends are tied off individually, in particular knotted in a self-contained matter.

16. Vessel prosthesis according to one of the preceding claims, in particular according to Claim 15, **characterized in that** the longitudinal seams have, between the thread ends, knots (12, 16, 28, 29).

17. Vessel prosthesis according to one of the preceding claims, **characterized in that** the longitudinal seams have, between the thread ends, at every one to three pleat folds, intermediate ties, in particular knots (12, 16, 28, 29).

18. Vessel prosthesis according to one of the preceding claims, **characterized in that** in each case 2 to 6, in particular in each case 2 to 3, folds (3) are gathered together by a transverse seam.

19. Vessel prosthesis according to one of the preceding claims, **characterized in that** it has several, in particular two, longitudinal seams (13a, 13b, 17a, 17b) which are preferably substantially parallel and run across a multiplicity of pleat folds (3).

20. Vessel prosthesis according to one of the preceding claims, **characterized in that** the folds (3) of the pleating, for fixing the arc, are fixed in a position close to one another on the inner side of the arc by at least one extension-resistant strip (31) secured thereon.

21. Vessel prosthesis according to Claim 20, **characterized in that** the strip (31) is secured at least on, in particular only on, the fold peaks, in particular adhesively bonded onto these.

## Revendications

1. Prothèse de vaisseau sanguin (1, 26, 30), en particulier pour remplacer des zones aortiques proches du coeur, sous la forme d'un tube flexible avec un plissage présentant des plis (3), dans laquelle le tube se présente en forme d'arc et la forme d'arc, à l'état non implanté, est fixée de façon résistante à la traction par au moins une limitation de traction continue s'étendant en direction longitudinale et/ou par une pluralité de limitations de traction s'étendant en direction transversale, dans laquelle les plis du plissage sont serrés pour la fixation de l'arc, **caractérisée en ce que** la prothèse de vaisseau sanguin présente comme limitation de traction au moins une couture (7, 13a, 13b, 17a, 17b, 19, 23, 24, 25) avec au moins huit piqûres (6), dans laquelle ladite au moins une couture s'étend en direction longitudinale et/ou périphérique de la prothèse, des extrémités de fils sont disposées à distance l'une de l'autre et des coutures longitudinales présentent des attaches intermédiaires entre les extrémités de fils.

2. Prothèse de vaisseau sanguin selon la revendication 1, **caractérisée en ce que** le tube plissé se présente sous la forme d'un arc préfixé thermiquement.

3. Prothèse de vaisseau sanguin selon l'une quelconque des revendications 1 ou 2, **caractérisé en ce que** des plis (3) du plissage sont serrés pour la fixation de l'arc et le serrage s'étend sur une partie de la périphérie du tube qui vaut de 10 à 180°.

4. Prothèse de vaisseau sanguin selon l'une quelconque des revendications précédentes, **caractérisée en ce qu'**elle est réalisée de façon résistante à la traction au moins sur le côté interne (4) de l'arc.

5. Prothèse de vaisseau sanguin selon l'une quelconque des revendications précédentes, **caractérisée en ce qu'**au moins une couture (7, 23) se trouve sur une ligne centrale (9) du côté interne (4) de l'arc.

6. Prothèse de vaisseau sanguin selon l'une quelconque des revendications précédentes, **caractérisée en ce qu'**au moins une couture (7, 13a, 13b, 17a, 17b, 19) présente des points de croix (8, 14, 18, 20), en particulier se compose de tels points.

7. Prothèse de vaisseau sanguin selon l'une quelconque des revendications précédentes, **caractérisée en ce qu'**au moins une couture, en particulier au moins une couture longitudinale, présente entre environ 1 et 40, de préférence entre 20 et 30, points, de préférence points de croix (8, 14, 18, 20) par couture.

8. Prothèse de vaisseau sanguin selon l'une quelconque des revendications précédentes, **caractérisée en ce que** des coutures, en particulier des coutures (7, 13a, 13b, 17a, 17b, 19) présentant des points de croix (8, 14, 18, 20), s'étendent dans la direction périphérique de la prothèse, en particulier de façon sensiblement parallèle l'une à l'autre, dans laquelle elles s'étendent de préférence sur une largeur qui correspond à environ 5 - 50 %, en particulier environ 10 à 40 %, de la périphérie totale du tube.

9. Prothèse de vaisseau sanguin selon l'une quelconque des revendications précédentes, **caractérisée en ce que** des coutures (7, 13a, 13b, 17a, 17b, 19) avec des points de croix (8, 14, 18, 20) s'étendent en direction longitudinale et/ou en direction transversale de la prothèse.

10. Prothèse de vaisseau sanguin selon l'une quelconque des revendications précédentes, **caractérisée en ce qu'**au moins une couture (23, 24, 25) présente des points de surjet (22) en particulier se compose exclusivement de tels points.

11. Prothèse de vaisseau sanguin selon l'une quelconque des revendications précédentes, **caractérisée en ce que** des coutures (23, 24, 25) avec des points de surjet (22) s'étendent en direction périphérique de la prothèse.

12. Prothèse de vaisseau sanguin selon l'une quelconque des revendications précédentes, **caractérisée en ce qu'**au moins une couture présente des points de piqûre, en particulier se compose exclusivement de tels points.

13. Prothèse de vaisseau sanguin selon l'une quelconque des revendications précédentes, **caractérisée en ce que** les bords intérieurs des plis (3) du plissage sont libres de limitations de traction, en particulier de fils.

14. Prothèse de vaisseau sanguin selon l'une quelconque des revendications précédentes, **caractérisée en ce qu'**au moins une couture (7, 13a, 13b, 17a, 17b, 19) présente au moins un fil traversant les plis (3) du plissage et s'étendant sur toute la longueur du serrage.

15. Prothèse de vaisseau sanguin selon l'une quelconque des revendications précédentes, **caractérisée en ce que** les extrémités de fils sont liées individuellement, en particulier sont nouées sur elles-mêmes.

16. Prothèse de vaisseau sanguin selon l'une quelconque des revendications précédentes, en particulier selon la revendication 15, **caractérisée en ce que** les coutures longitudinales présentent des noeuds (12, 16, 28, 29) entre les extrémités de fils.

17. Prothèse de vaisseau sanguin selon l'une quelconque des revendications précédentes, **caractérisée en ce que** les coutures longitudinales présentent des attaches intermédiaires, en particulier des noeuds (12, 16, 28, 29) entre les extrémités de fils tous les un à trois plis de plissage.

18. Prothèse de vaisseau sanguin selon l'une quelconque des revendications précédentes, **caractérisée en ce que** chaque fois 2 à 6, en particulier chaque fois 2 à 3, plis (3) sont serrés par une couture transversale.

19. Prothèse de vaisseau sanguin selon l'une quelconque des revendications précédentes, **caractérisée en ce qu'**elle présente plusieurs, en particulier deux, coutures longitudinales (13a, 13b, 17a, 17b) de préférence essentiellement parallèles, qui s'étendent sur une pluralité de plis de plissage (3).

20. Prothèse de vaisseau sanguin selon l'une quelconque des revendications précédentes, **caractérisée en ce que** les plis (3) du plissage sont fixés en position rapprochée l'un de l'autre au moyen d'au moins une bande (31) résistant à la traction fixée à ceux-ci sur le côté interne de l'arc pour la fixation de l'arc.

21. Prothèse de vaisseau sanguin selon la revendication 20, **caractérisée en ce que** la bande (31) est fixée au moins sur, et en particulier uniquement sur les pointes des plis, en particulier est collée sur celles-ci.
